# EUROPEAN PATENT APPLICATION

(11) **EP 4 595 865 A1**
(43) Date of publication of application: **06.08.2025**
(21) Application number: 24154700.9
(22) Date of filing: 30.01.2024
(51) Int. Cl.: A61B 1/018, A61B 1/05, A61B 1/06, G02B 23/24

(54) **ENDOSCOPE TIP PART WITH UNIVERSAL CAMERA ASSEMBLY**

(71) Applicant: Ambu A/S, 2750 Ballerup (DK)
(72) Inventor: SEIBÆK, Mikkel Haugaard, 2400 København NV (DK); RASK, Jesper Domino, 2300 København S (DK); HANSEN, Frederik Clausager Vemb, 2400 København NV (DK)
(74) Representative: Winter, Brandl - Partnerschaft mbB

(57) **Abstract**

An endoscope including a positioning interface or handle; an insertion tube extending distally from the positioning interface or handle; a lighting module including a body having a longitudinal bed and a transverse wall, a light emitting diode supported at a distal end of the longitudinal bed, and lighting wires electrically connected to the light emitting diode and extending through the insertion tube to the positioning interface or handle; a universal camera module comprising an image sensor, a circuit board electrically connected to the image sensor, a sensor holder, and a sensor housing affixed to the sensor holder, the image sensor housed in the sensor housing, and the sensor holder extending proximally from the image sensor.

## Description

### CROSS-REFERENCE TO RELATED APPLICATIONS

None.

The present application is related to U.S. Patent Application No. (docket no. 1256) and U.S. Patent Application No. (docket no. 1272).

### TECHNICAL FIELD

The present disclosure relates to an endoscope tip assembly, an endoscope with said tip assembly, a method for manufacturing said tip assembly, and a manufacturing system for the tip assembly.

### BACKGROUND

Endoscopes are well-known devices in the medical field for visually examining the interior of a patient. An endoscope generally includes a handle or positioning interface and an insertion cord extending distally therefrom. The insertion cord includes an insertion tube, a bending section, and a distal tip. The distal tip comprises a housing accommodating a camera assembly including an image sensor. The following commonly-owned U.S. Patents and Publications, incorporated herein by reference, disclose examples of endoscopes with a variety of distal tips: U.S. Patents Nos. 11,291,352; 11,311,184; 11,432,714; 11,642,010; and U.S. Patent Publications Nos. 20200281445; 20200288953; 20200405137; 20210068640; 20210068641; 20220061645; 20220175226 and 20230054149.

Single-use endoscopes optimize workflow and reduce cost while saving patient's lives and improving patient care. They optimize workflow and reduce cost because they are always ready when needed without the traditional large-scale capital and repair budgets required for reusable endoscopes. For example, a sterilization and storage facility is avoided, there is no need to maintain evidence of sterilization, and there is no need to transport endoscopes from sterilization and storage facilities to the buildings where they are needed, sometimes in the middle of the night or weekends. They save patient's lives and improve patient care because they are readily available and do not pose a cross-contamination risk. This also reduces hospital readmissions. While single-use endoscopes are disposed after a single patient use (one or more procedures may be performed while the patient remains in the treatment room), the environmental impact of re-useable endoscopes, due to cleaning materials, CO₂ emissions during the cleaning process, and use of disposable personal protective equipment by personnel involved in transportation and sterilization of the re-useable endoscopes, can be similar to that of single-use endoscopes. To further reduce environmental impact, the endoscopes according to the present disclosure are primarily made of polymer materials.

Endoscopes, both reusable and disposable (i.e. single-use), include procedure-specialized endoscopes, for example arthroscopes, bronchoscopes, cholangioscopes, colonoscopes, cystoscopes, duodenoscopes, gastroscopes, laparoscopes, ureteroscopes, and others. To manufacture procedure-specialized endoscopes a supply-chain is necessary for the production of various components, such as image sensors, light sources, steering wires, working channel tubes, and the like. The product-mix and inventory investment of the supply-chain grows further when endoscopes are produced in different sizes, adding to the product mix.

To further enhance the benefits of single-use endoscopes, it is desirable to reduce manufacturing costs.

### SUMMARY

The present invention provides a universal camera module design. The dimensions and design of the universal camera module are kept constant no matter the size, particularly of the outer diameter, of the distal tip housing and endoscope.

The universal camera module design can be used and implemented across a wide range of endoscopes with or without working channels, thus allowing a single camera module to be mass produced and used across a range of endoscopes. A single type of injection molds and tooling are needed. The universal camera module design thus reduces design and supply-chain costs, including work-in-process inventory. Furthermore, for the different endoscope types, less time needs to be spent designing the camera compartment in the tip housing, as the universal camera module dimensions are already fixed. Similarly, time spent on electronic implementation (e.g. image processing) of the universal camera module in a new endoscope is hugely reduced as the same universal camera module and components are used.

This is achieved by providing a tip housing and universal camera module with a geometry that provides surfaces for guiding and securing the universal camera module in both the smallest outer diameter endoscopes as well as the largest outer diameter endoscopes. Furthermore, by splitting the camera and lighting modules into separate modules (including separate printed circuit boards (PCBs) and separate wiring) the design also allows for different lighting configurations to be used depending on the size and type of endoscope. In larger endoscopes and where more free space is available, more, or larger light emitting diodes (LEDs) can be used. Optical fibers and light guides can also be used in the lighting module.

A first aspect of the invention disclosed herein is to reduce manufacturing costs by designing endoscopes, including procedure-specialized endoscopes, with common parts. Utilization of common parts simplifies the supply-chain and thus reduces inventory costs, such as the cost to maintain large inventories and the obsolescence cost resulting from product improvements.

In an embodiment according to the first aspect, a system is provided, comprising: two copies of a universal camera module; a first lighting module; a second lighting module; a first tip housing devoid of a working channel; and a second tip housing comprising a working channel, wherein the first tip housing is sized and configured to receive one copy of the universal camera module and the first lighting module, and wherein the second tip housing is sized and configured to receive the second copy of the universal camera module and the second lighting module, and wherein the first tip housing, the universal camera module and the first lighting module form a first tip assembly, wherein the second tip housing, the universal camera module and the second lighting module form a second tip assembly.

In another embodiment according to the first aspect, an endoscope comprises a positioning interface or handle; an insertion tube extending distally from the positioning interface or handle; a lighting module comprising a body including a longitudinal bed and a transverse wall, a light emitting diode supported at a distal end of the longitudinal bed, and lighting wires electrically connected to the light emitting diode and extending through the insertion tube to the positioning interface or handle; a universal camera module comprising an image sensor, a circuit board electrically connected to the image sensor, a sensor holder, and a sensor housing affixed to the sensor holder, the image sensor housed in the sensor housing, and the sensor holder extending proximally from the image sensor; camera module wires electrically connected to the circuit board electrically connected to the image sensor, the camera module wires extending through the insertion tube to the positioning interface or handle; and a tip housing enclosing the lighting module and the universal camera module.

A second aspect of the invention disclosed herein is to provide a method of manufacturing endoscopes which simplifies the assembly process.

In an embodiment according to the second aspect, the method comprises: assemblying a first copy of a universal camera module and a second copy of the universal camera module, the first copy being identical to the second copy; assembling a first lighting module; assembling a second lighting module different than the first lighting module; providing a first tip housing; providing a second tip housing different than the first tip housing; inserting one copy of the universal camera module and the first lighting module into the first tip housing to form a first tip assembly; and inserting the second copy of the universal camera module and the second lighting module into the second tip housing to form a second tip assembly.

A third aspect is to provide an endoscope with an improved working channel tube connection at the tip of the endoscope.

In an embodiment according to the third aspect, an endoscope comprises a tip assembly comprising; a lighting module comprising a light emitting diode; a universal camera module comprising an image sensor, a circuit board electrically connected to the image sensor, and a sensor housing, the image sensor housed in the sensor housing; and a tip housing enclosing the lighting module and the universal camera module, the tip housing comprising a working channel, a distal portion, a transition piece and a middle portion positioned between the distal portion and the transition piece, the middle portion being opaque and the distal portion being transparent, the working channel comprising a middle portion working channel and a transition piece working channel, wherein the middle portion comprises the middle portion working channel, and wherein the transition piece comprises the transition piece working channel, the transition piece working channel including a distal working channel portion distal of a proximal working channel portion, the proximal working channel portion being offset from the middle portion working channel. The tip housing may also comprise a proximal portion, in which case the middle portion is positioned between the distal portion and the proximal portion, with a separate transition piece affixed to the proximal portion. The proximal portion may be transparent and molded in one piece with the middle portion and the distal portion.

In a variation of the present embodiment, the middle portion working channel comprises a longitudinal axis WCA, and the proximal working channel portion comprises a longitudinal axis TPA(p) that is parallel to the longitudinal axis of the middle portion working channel.

In another variation, the tip housing comprises a camera module cavity, the transition piece comprises a distal protrusion that fits in the camera module cavity and a proximal joint surface, and the the middle portion comprises a distal joint surface adhesively bonded to the proximal joint surface forming a joint, the distal protrusion extending distally of the joint.

In a further variation, the middle portion comprises a circumferential wall having an arcuate portion, a distal joint surface, the transition piece comprising a proximal joint surface and a circumferential wall portion positioned anteriorly of the transition piece working channel, the distal joint surface being adhesively bonded to the proximal joint surface forming a joint, and the arcuate portion extending over the joint.

As used herein, "anterior" refers to the side of the universal camera module that is opposite the lighting module and the term "posterior" refers to the side of the universal camera module that is adjacent the lighting module and, when present, adjacent a working channel. The word anterior could be substituted for the words top or upper, and the word posterior could be substituted for the words bottom or lower.

One or more of these objects may be met by aspects of the present invention described in the following embodiments, variations and examples thereof.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

The invention will be described in more detail below with reference to the following figures. The figures illustrate embodiments, variations and examples of the invention to facilitate the understanding of a person of ordinary skill in the art and are not to be construed as limiting the scope of the invention.
FIG. 1 is a perspective illustration of a visualization system including an endoscope and a video processor connectable to the endoscope;
FIG. 2 is a perspective illustration of an embodiment of a universal camera module;
FIG. 3 is a perspective illustration of a first embodiment of a lighting module;
FIG. 4 is a perspective illustration of a first embodiment of a tip assembly comprising a first embodiment of a tip housing and including the lighting module of FIG. 3 and the universal camera module of FIG. 2;
FIG. 5 is a perspective illustration of a second embodiment of a lighting module;
FIG. 6 is a perspective illustration of a second embodiment of a tip assembly comprising a second embodiment of a tip housing and including the lighting module of FIG. 5 and the universal camera module of FIG. 2;
FIG. 7 is a perspective illustration of the embodiment of the universal camera module of FIG. 2;
FIG. 8 is a perspective illustration of the embodiment of the lighting module of FIG. 3;
FIG. 9 is a lateral view of the lighting module of FIG. 3 and the universal camera module of FIG. 2;
FIG. 10 is a lateral of the first embodiment of the tip assembly of FIG. 4;
FIG. 11 is a perspective view of the tip assembly of FIG. 4;
FIG. 12 is a perspective sectional view of the tip assembly of FIG. 4;
FIG. 13 is a proximal view of the first embodiment of the tip housing;
FIG. 14 is a proximal view of the second embodiment of the tip housing;
FIG. 15 is a proximal perspective view of the second embodiment of the tip housing;
FIG. 16 is a perspective view of the tip assembly of FIG. 6;
FIG. 17 is a perspective sectional view of the tip assembly of FIG. 6;
FIG. 18 is a lateral sectional view of a first embodiment of a transition piece of the second embodiment of the tip housing;
FIGS. 19 and 20 are perspective views of components of the second embodiment of the tip housing;
FIGS. 21 and 22 are perspective views of components of another example of the second embodiment of the tip housing;
FIGS. 23 and 24 are perspective views of the second lighting module;
FIG. 25 is a flowchart depicting an embodiment of a method of making endoscopes using the universal camera modules and lighting modules of FIGS. 2 to 23;
FIG. 26 is a schematic view of a light guide; and
FIGS. 27 and 28 are views of examples of video processors.

### DETAILED DESCRIPTION OF THE INVENTION

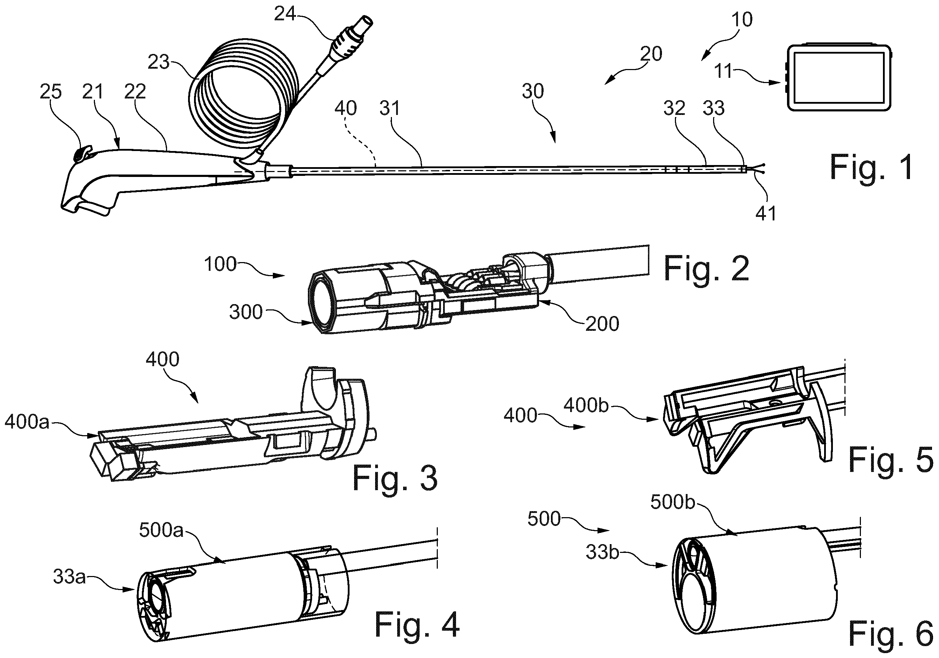

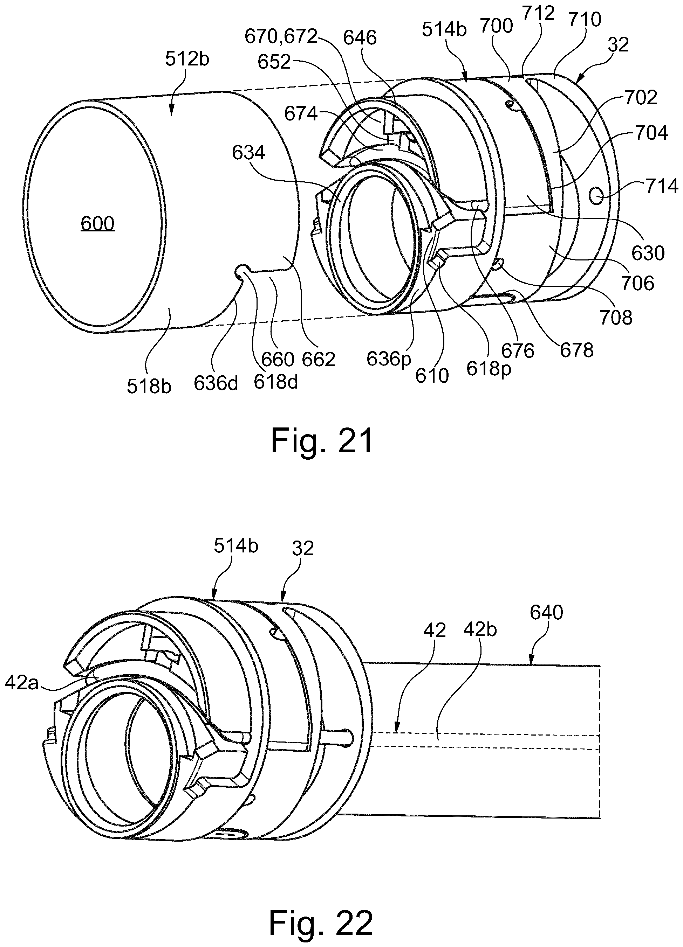

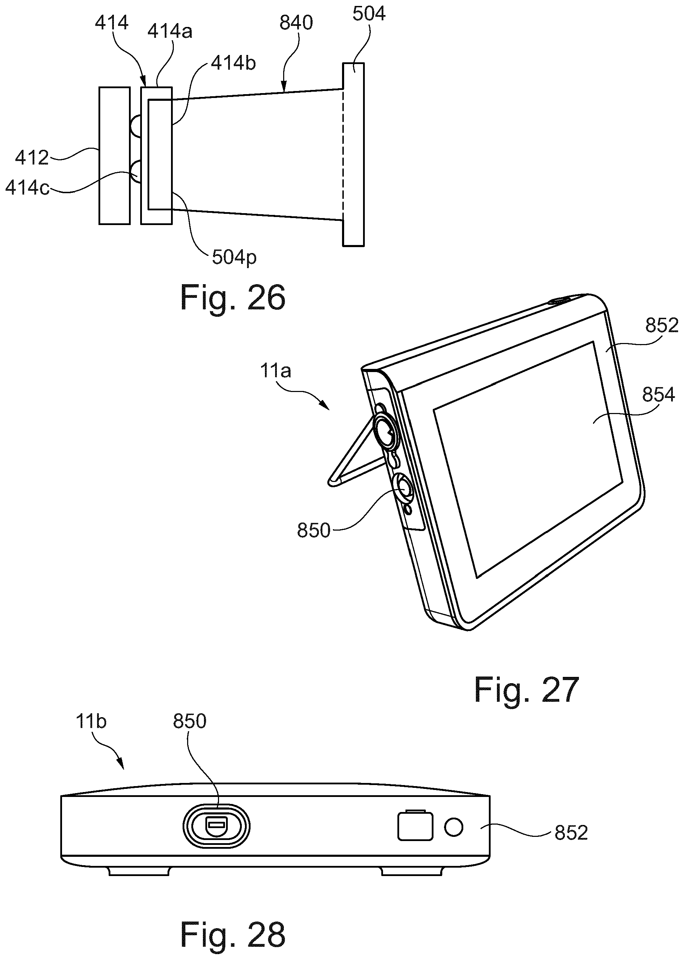

FIG. 1 illustrates an embodiment of a visualization system 10 including a video processor 11 and an endoscope 20 including a universal camera module. The endoscope 20 comprises a handle 21 comprising a housing 22 and a steering actuator 25, illustratively a rotatable lever or wheel. A wire cord 23 includes a connector 24 that is receivable by one of the connector receptacles of the video processor 11 to establish electronic communications between a respective medical device interface of the video processor 11 and the endoscope 20. The endoscope 20 also comprises an insertion cord 30 including an insertion tube 31, a bending section 32, and a tip assembly 33. In the present embodiment, the endoscope 20 comprises a working channel 40. A tool 41 is shown extending through the insertion cord 30, through the working channel 40, and out the tip assembly 33. A steering wire 42 (shown in FIG. 22) is operably connected to the steering actuator 25 and configured to steer the tip assembly 33. Embodiments of the video processor 11 are described with reference to FIGS. 26 and 27.

The universal camera module and a system to reduce manufacturing costs will now be described with reference to FIGS. 2 to 6. FIGS. 2 to 6 illustrate two embodiments of the tip assembly 33, both embodiments comprising a universal camera module 100 (FIG. 2) including a sensor holder 200 and a sensor housing 300. FIGS. 3 and 5 show embodiments of a lighting module 400, denoted by numerals 400a and 400b, of first and second lighting modules. FIGS. 4 and 6 show embodiments of the tip assembly 33, denoted by numerals 33a and 33b, each including the universal camera module 100. The first tip assembly 33a includes the first lighting module 400a and a first tip housing 500a, and the second tip assembly 33b includes the second lighting module 400b and a second tip housing 500b different from the first tip housing 500a. The first and second lighting modules 400a, 400b are variants of a lighting module 400 and the first and second tip housings 500a, 500b are variants of a tip housing 500. As shown, the tip assemblies are assembled with different lighting modules and tip housings using the same universal camera module 100 design, which simplifies design and manufacturing while reducing work-in-progress and supply-chain inventory. Components of parts or subassemblies denoted by numerals 100, 200, 300, 400 and 500 will be described below in sequences beginning, respectively with numerals 102, 202, 302, 402, and 502.

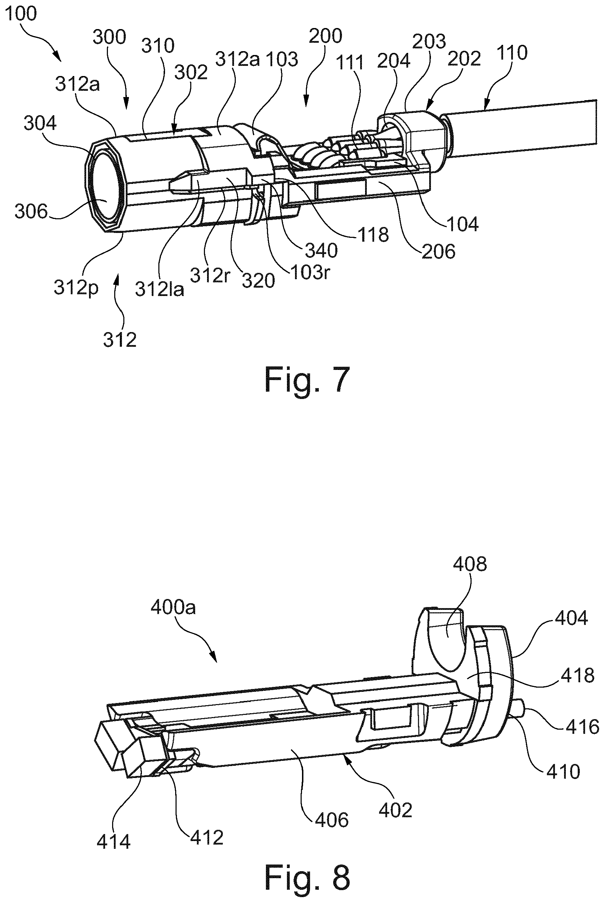

In some embodiments, when assembled in endoscope tip housings, with or without a working channel, the front surface of the sensor housing 300 may abut an inner proximal surface of the tip housing 500a, 500b restricting movement in the proximal-distal direction. The front surface may be a surface at the distal end of the tip housing that is transverse to the longitudinal extent of the tip housing. Anterior and posterior distal surfaces of the sensor housing 300 abut corresponding anterior and posterior distal inner surfaces of the tip housing, thereby restricting anterior/posterior movement of the distal end of the sensor housing 300. To restrict lateral movement of the sensor housing 300, surfaces of guide, or alignment, "wings" on each side of the sensor housing 300 abut corresponding guide or alignment surfaces in the tip housing 500a, 500b. Tip housings with working channels and without working channels differ in how the proximal end of the sensor housing 300 is supported and how rotational movement of the sensor housing 300 is restricted, as explained with reference to FIG. 7.

The dimensions of the universal camera module 100, particularly the outer dimensions, may be fixed so that the universal camera module 100 may be used with tip housings of different dimensions, with and without working channels. Advantageously, fixed dimensions enable use of the same camera module across a wide range of endoscopes, thus allowing a single camera module to be mass produced and used across a range of endoscopes or, more broadly, insertable visualization devices, including endotracheal tubes. Thereby, a single set of injection molds and tooling is needed to manufacture the universal camera module 100. Furthermore, for the different endoscope types, less time needs to be spent designing the camera compartment inner cavity in the tip housing, as the universal camera module dimensions are already fixed. Similarly, time spent on electronic implementation (e.g. image processing) of the universal camera module in a new endoscope is hugely reduced as the same universal camera module and components are used.

FIG. 7 is a perspective view of an embodiment of the universal camera module 100 including the sensor holder 200 and the sensor housing 300, which are affixed at joints 118. The universal camera module 100 is very small. In some embodiments, the universal camera module 100 is about the size of a grain of rice, having a cross-section smaller than 2 mm, potentially smaller than 1.5 mm in its largest extent. The largest extent of the cross-section of the tip housing 500 may be 3 mm, preferably less than 2.8 mm, and even more preferred less than 2.7 mm in endoscopes without working channels. Clearly, assembling multiple parts into a universal camera module 100 of such small proportions benefits from innovative assembly methods. Such methods, described below, are also beneficial when manufacturing the universal camera module 100 with primarily plastic parts (except for the electronic components, such as the image sensor, circuit boards, and LEDs) to make a single-use tip assembly.

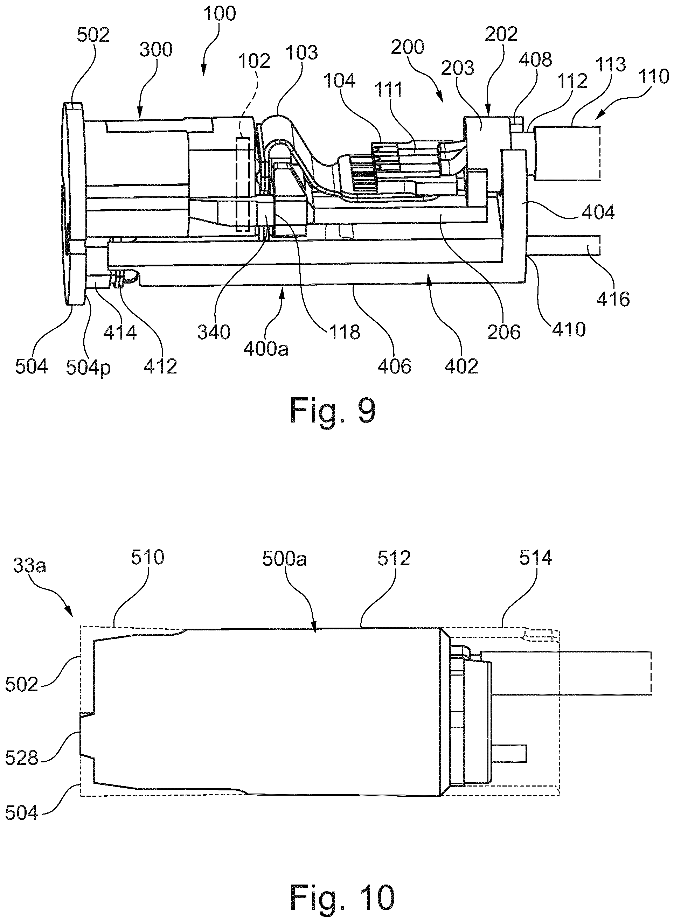

In the present embodiment, the universal camera module 100 comprises a circuit board 103 electrically connected to an image sensor and the camera module wires 111. The camera module wires 111 may form, together with an optional shield and/or cover, a cable assembly 110. Optionally, a circuit board 104 may be electrically connected to the circuit board 103. Use of the circuit board 104 facilitates soldering of the camera module wires 111 before assembly of the universal camera module 100 by enabling simple soldering or connecting of two flat surfaces of the circuit boards 103, 104. The present embodiment of the universal camera module 100 further comprises, as shown in FIG. 9, an image sensor 102, an electrical shield 112, and a cable cover 113. The cable assembly 110 comprises the camera module wires 111, the electrical shield 112, and the cable cover 113. Each of the joints 118 is preferably formed by adhesive bonding. The circuit board 103 may comprise a flexible printed circuit board. The image sensor 102 may be electrically connected to the circuit board 103, the circuit board 104, and the camera module wires 111, before assembly of these components onto the sensor holder 200. Then, the sensor holder 200 may be affixed to the sensor housing 300 after placement of the image sensor 102 in a sensor cavity of the sensor housing 300.

The sensor holder 200 comprises a cable holder 202 including an outer surface 203 and a recess 204. The recess 204 is, optionally, a through-hole. The cable holder 202 is connected to a longitudinal bed 206 including a bed surface to which the circuit boards may be attached. The camera module wires 111 pass through the recess 204. At a distal end of the longitudinal bed 206 a distal surface forms the joint 118 with an opposing surface of the sensor housing 300.

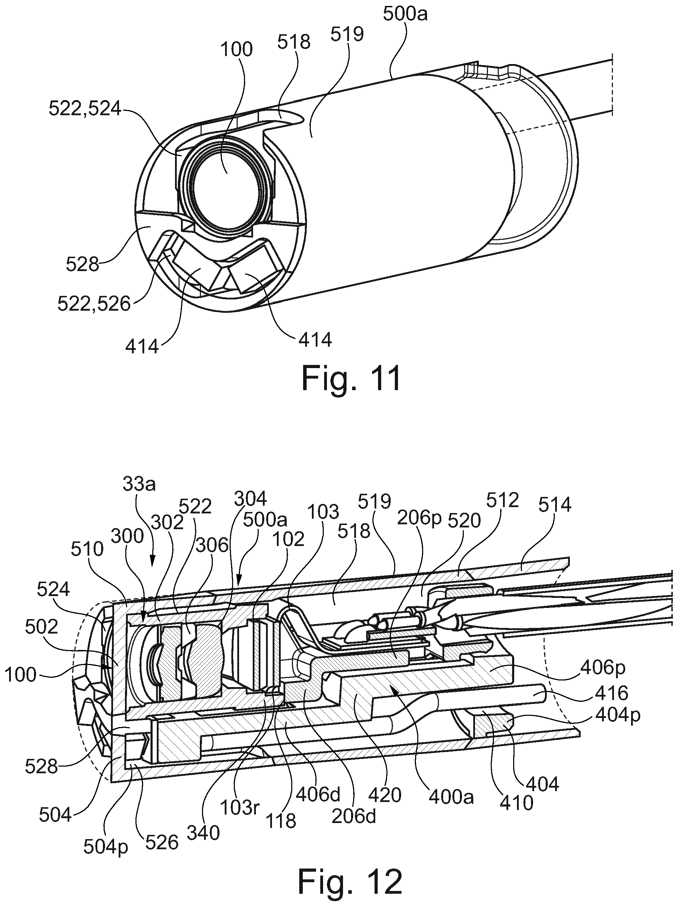

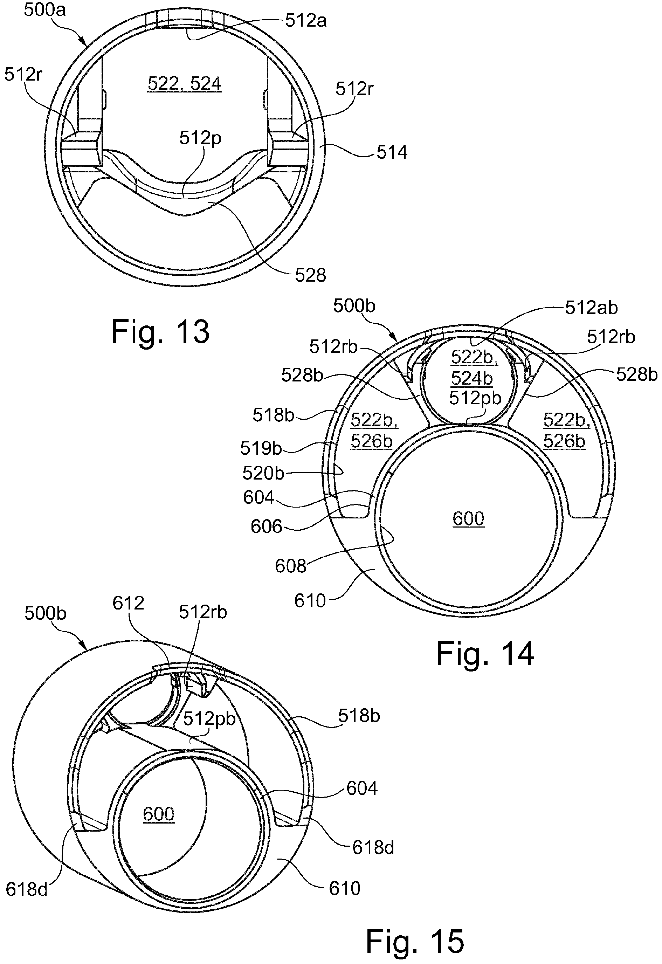

The sensor housing 300 comprises a circumferential wall 302 that defines a sensor cavity 304 in which lenses 306 (shown in FIG. 12) and the image sensor 102 are housed, the lenses 306 positioned distally of the image sensor 102. The circumferential wall 302 has an outer surface 310. The sensor housing 300 is opaque to block stray light from entering the sensor cavity 304 and potentially distorting the desired images captured by the image sensor 102. Arms 340 extend proximally, and wings 320 extend laterally, from the circumferential wall 302. Alignment surfaces 312 are provided, denoted as surfaces 312a, 312p, 312la and 312r, on the outer surface 310 and/or the arms 340 and/or the wings 320, which contact opposed alignment surfaces of the tip housing 500, 500a, 500b. More than one posterior alignment surfaces 312p and anterior alignment surfaces 312a may be provided. Alignment surfaces 312r, 312ia prevent rotational movement and may be referred to as "anti-rotation" surfaces. The anti-rotation surfaces, as shown, are surfaces of the wings. Alternatively, they may be surfaces of the sensor holder. The sensor holder may comprise the wings 320. The sensor holder and the sensor housing may comprise the wings 320. The anterior alignment surfaces 312a are portions of the outer surface 310. The alignment surfaces 312 abut corresponding surfaces of the tip housing 500 to limit anterior/posterior and rotational movement of the universal camera module 100 in the tip housing 500, 500a, 500b. As shown, the anti-rotation surfaces 312r are posterior surfaces of the wings 320. Alternatively, the anti-rotation surfaces 312r may be posterior surfaces of the arms 340. The anti-rotation surfaces 3121a may be lateral surfaces of the wings 320 or the arms 340 and are provided to abut with alignment surfaces 512rb of the tip housing 500b, which are shown in FIG. 14.

In one variation, two posterior alignment surfaces 312r and one anterior alignment surface 312a are provided. In the present variation, the tip housing 500a may be cantilevered on the anti-rotation surfaces 312r, which are located proximally of the anterior alignment surface 312a, to precisely place and align the sensor housing 300 in the tip housing 500a in a simple, thus economical, manner.

The arms 340 may extend through holes or recesses/cut-outs 103r in the circuit board 103. Either or both of the sensor holder 200 and the sensor housing 300 may comprise one or more of the arms 340 that extend to form the joint 118 with a portion of the circuit board 103 therebetween. Three arms 340 are preferred because three arms provide sufficient structural support while requiring less space than more than three arms. The circuit board 103 may comprise a flexible circuit board connected electrically with a rigid circuit board that is affixed to the image sensor 102.

Referring now to FIG. 8, the first lighting module 400a comprises a body 402 including a proximal transverse wall 404 and a longitudinal bed 406 extending distally from the proximal transverse wall 404. The proximal transverse wall 404 comprises a cable recess 408 configured to receive the cable assembly 110 (for the camera) or the wires thereof and/or the cable holder 203. At a distal end, the longitudinal bed 406 supports light emitting diodes (LEDs) 414 electrically connected to a circuit board 412. Electrical lighting wires 416 electrically connect the LEDs 414, via the circuit board 412, to a power source at the handle or at the video processor to power the LEDs. The proximal transverse wall 404 also comprises a cable recess 410 configured to receive the lighting wires 416 therethrough. As shown, the cable recess 408 is a cut-out and the cable recess 410 is a through-hole separated from the cable recess 408 by an intermediate portion 418 of the transverse wall 404, and the longitudinal bed 406 extends longitudinally along a plane traversing the intermediate portion of the transverse wall 404. The cable holder 203 may be translated longitudinally into the cable recess 408 or may slid through the open end of the cut-out. Alternatively, the cable recess 408 may be a through-hole and, thus, the cable holder 203 may be translated longitudinally into the cable recess 408.

The first lighting module 400a may be electrically isolated, at the tip assembly, from the universal camera module 100. By electrically isolated it is meant that there is no electrical connection between first lighting module 400a and the universal camera module 100. Isolation also enables independent control of the light intensity, which can be varied in different endoscopes without requiring changes to the camera module, thus rendering universal. Lighting may be optimized for each endoscope by selecting different light emitters and modifying configurations of the light emitters, for example by optimizing size, color, light intensity, position and orientation. Some endoscopes may comprise light emitters that emit light at different frequencies. Light emitters may comprise LEDs and/or light fibers extending through the insertion cord and receiving light from a source located at the handle or a separate light source. Alternatively, the lighting wires may be connected to the circuit board 103 or 104.

FIG. 9 illustrates how the universal camera module 100 and the first lighting module 400a are aligned, in one embodiment, before they are inserted into the tip housing 500a. The universal camera module 100 is not necessarily affixed to the first lighting module 400a and, therefore, can move, relative to the first lighting module 400a, in the anterior/posterior direction, longitudinally, and/or rotatably, so that alignment of the universal camera module 100 in the tip housing 500a is independent of the alignment of the first lighting module 400a in the tip housing 500a.

Also shown in FIG. 9 are a viewing window 502 and lighting windows 504, which may be part of the tip housing 500a. The viewing window 502 and the lighting windows 504 may be molded in a one-piece part with the tip housing 500a to form a sealed enclosure for the universal camera module 100. During assembly, distal movement of the first lighting module 400a stops when the LED 414 abuts the lighting window 504. Alternatively, a light guide may extend proximally from the lighting window 504 and in that case distal movement of the first lighting module 400a stops when the LED 414 abuts the light guide. More generally, the light emitted by the light source is received by a light receiving surface, which can be the proximal surface of the light guide or the proximal surface of the lighting window, and is emitted by a distal surface of the lighting window. Example light guides are discussed with reference to FIG. 26. The viewing window 502 and lighting windows 504 may, alternatively, be attached to the sensor housing 300 or the tip housing 500a.

Referring now to FIGS. 10 and 11, the tip housing 500a comprises a distal portion 510, a middle portion 512, and a proximal portion 514. The proximal portion 514 and the distal portion 510 may be transparent while the middle portion 512 may be opaque. The distal portion 510 comprises the viewing window 502 and the lighting windows 504, which are separated by an intermediate, opaque, wall 528 that is part of the middle portion 512. Transparent portions allow light to pass therethrough. In the proximal portion 514 passage of light facilitates ultraviolet curing of ultraviolet curable adhesives, which simplifies manufacturing and thus reduces costs. In the distal portion 510 the passage of light allows capturing of images by the image sensor 102 and illumination by the LEDs 414. The middle portion 512 comprises a circumferential wall 518 having a wall outer surface 519 and a wall inner surface 520 (see FIG. 12) that forms a camera module cavity 522 comprising a sensor module cavity 524 and an LED cavity 526. The middle portion 512 is molded with the intermediate wall 528. The circumferential wall 518 may be shaped as a cylinder although non-circular cross-sections are permissible. The sensor module cavity 524 is positioned on one side of the intermediate wall 528 and the LED cavity 526 is positioned on the other side of the intermediate wall 528.

Once the universal camera module 100 and the first lighting module 400a are positioned and aligned in the first tip housing 500a, an adhesive is inserted into the inner cavity to adhesively bond the universal camera module 100, the first lighting module 400a and the first tip housing 500a to each other (or to adhesively bond the universal camera module 100, the second lighting module 400b and the second tip housing 500b to each other), allowing the universal camera module 100 and the respective lighting module to be inserted into the respective tip housing with relative movement between the universal camera module 100 and the lighting module, so that positioning of the universal camera module 100 is independent of positioning of the lighting module, which provides the advantage that the chain of assembly tolerance is severed and the tip assembly can be assembled with less variation than if the universal camera module 100 were affixed to the lighting module prior to insertion into the tip housing. An assembly chain comprises multiple assembly steps performed in sequence. Typically, each step has an amount of variation, or tolerance, and performing the steps sequentially increases the variation of the final assembly because the variation of each step increases the variation in the immediately subsequent step. Severing the assembly tolerance chain results in a shorter sequence of steps that depend on each other, and thus results in a more precisely assembled tip assembly, with less variation and thus improved quality and performance.

FIG. 12 is a perspective section view of the tip assembly 33a with the universal camera module 100 positioned in the tip housing 500a. The longitudinal bed 206 of the sensor holder 200 may comprise a distal bed portion 206d that is offset relative to a proximal bed portion 206p, Similarly, the longitudinal bed 406 of the lighting module 400 may comprise a distal bed portion 406d that is offset relative to a proximal bed portion 406p. In this context offset means that the distal portion is displaced posteriorly from the proximal portion, while both portions extend longitudinally in parallel. The offset creates a step 420 that creates space for the posterior side 404p of the transverse wall 404 and enables a smaller camera module. Thusly, the posterior side 404p is offset anteriorly relative to the distal bed portion 406d.

Referring now to FIG. 13, the tip housing 500a comprises at least three alignment surfaces, denoted as 512a, 512p and 512r, which may abut the alignment surfaces 312a, 312p, and 312r of the sensor housing 300. The posterior alignment surface 512p is a middle surface of the intermediate wall 528. Alignment surfaces 512r may be referred to as anti-rotation surfaces and are surfaces of the intermediate wall 528 positioned adjacent the circumferential wall 518. The anti-rotation surfaces surfaces 512r may extend along a transverse plane (transverse to an anterior/posterior plane) facing anteriorly. The anterior alignment surface 512a is an anterior portion of the wall inner surface 520 of the middle portion 512 of the tip housing 500a. As discussed above, the alignment surfaces of the tip housing 500a abut the alignment surfaces of the universal camera module 100 to allow securement of the universal camera module 100 in position independently of the first lighting module 400a. Alternatively, the universal camera module 100 may be mounted onto the first lighting module 400a such that the alignment surfaces allow securement of the universal camera module 100 and the first lighting module 400a in the precise desired position.

In a variation of the present embodiment, the at least three alignment surfaces consist of two anti-rotation surfaces 512r and one anterior alignment surface 512a.

In another variation of the present embodiment, the at least three alignment surfaces consist of two anti-rotation surfaces surfaces 512r and two anterior alignment surfaces 512a, one positioned distally of the other.

In a further variation of the present embodiment, the at least three alignment surfaces consist of two anti-rotation surfaces 512r, two anterior alignment surfaces 512a, and at least one posterior alignment surface 512p.

In a yet further variation of the present embodiment, the at least three alignment surfaces consist of two anti-rotation surfaces 512r, one anterior alignment surface 512a, and one posterior alignment surface 512p. The alignment surfaces 512a and 512p are positioned distally of the two anti-rotation surfaces 512r.

More generally, the anti-rotation surfaces can be pairs of surfaces positioned on opposite sides (laterally) of the universal camera module. Even more generally, only one anti-rotation surface may be provided. An example of an anti-rotation surface may be the surface of a protrusion that fits into a longitudinal slot of the tip housing. Another example of an anti-rotation surface may be the surface of a longitudinal slot into which a protrusion of the tip housing fits. The protrusion and slot of the universal camera module may comprise a curved surface and may be positioned in the sensor housing or the sensor holder.

Attention will now be turned to the second embodiment of the tip assembly, denoted as 33b, with reference to FIGS. 14 to 24. Parts of the tip assembly 33b comprising the suffix "b" are functionally equivalent of corresponding parts of the tip assembly 33a having the same prefix. Thus, the sensor housing 500a is functionally equivalent as the sensor housing 500b, although the sensor housing 500b provides additional functions. FIG. 14 is a proximal view of the tip housing 500b and FIG. 15 is a proximal perspective view of the middle portion 512 of the tip housing 500b. The tip housing 500b comprises at least three alignment surfaces 512b, denoted as 512ab, 512pb, and 512rb. The alignment surfaces 512rb are provided to prevent rotation of the universal camera module 100 and are oriented differently than the alignment surfaces 512r (these may be referred to as "anti-rotation" surfaces). As shown, the alignment surfaces 512rb are oriented facing each other and abut the lateral surfaces 3121a of the wings 340. Additionally, the tip housing 500b comprises a working channel 600 that forms part of the working channel 40. Accordingly, the universal camera module 100 comprises a first pair of anti-rotation surfaces 312r and a second pair of anti-rotation surfaces 312la, the tip housing 500a comprises a pair of anti-rotation surfaces 512r, and the tip housing 500b comprises a pair of anti-rotation surfaces 512rb.

The tip housing 500b comprises a working channel wall 604, a working channel wall outer surface 606, and a working channel wall inner surface 608. On the posterior side of the tip housing 500b the working channel wall inner surface 608 overlaps the wall surface 519b at a housing portion 610. The tip housing 500b also comprises intermediate walls 528b, and a circumferential wall 518b having a wall outer surface 519b and a wall inner surface 520b that forms, together with the working channel wall outer surface 606, a camera module cavity 522b comprising a sensor module cavity 524b and LED cavities 526b. The circumferential wall 518b may be shaped as a cylinder although non-circular cross-sections are permissible. The intermediate walls 528b are connected to and extend between the circumferential wall 518b and the working channel wall 604.

The circumferential wall 518b, as shown in FIG. 15, may comprise an anterior notch 612 and one or more inlet notches 618d. The inlet notches 618d match or are aligned with inlet notches 618p to form inlet holes 618 suitable for insertion of a liquid adhesive.

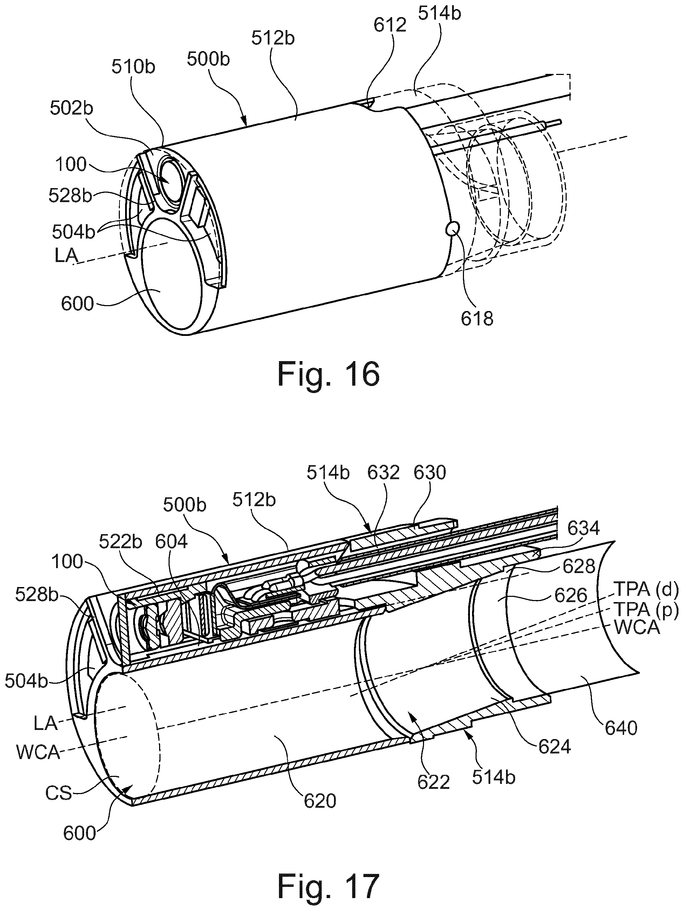

As seen in FIGS. 16 and 17, the tip housing 500b further comprises a distal portion 510b, a middle portion 512b, a proximal portion 514b. The proximal portion 514b may be referred to as the "transition piece". The proximal portion 514b and the transition piece 512b may be transparent while the middle portion 512b may be opaque. The distal portion 510b comprises the viewing window 502b and the lighting windows 504b, which are separated by the intermediate, opaque, walls 528b that are part of the middle portion 512b. The distal portion 510b may be molded with the middle portion 512b as a single-piece part. In the proximal portion 514b passage of light facilitates ultraviolet curing of ultraviolet curable adhesives, which simplifies manufacturing and thus reduces costs. In the distal portion 510b the passage of light allows capturing of images by the image sensor 102 and illumination by the LEDs 414. The middle portion 512b is molded with the intermediate walls 528b. The sensor module cavity 524b is positioned on one side of the intermediate walls 528b and the LED cavity 526b is positioned on the other side of the intermediate walls 528b. Alternatively, the tip housing may also comprise a proximal portion, in which case the middle portion is positioned between the distal portion and the proximal portion, with a separate transition piece affixed to the proximal portion. The proximal portion may be transparent and molded in one piece with the middle portion, as in the tip housing 500a shown in FIG. 12, and the transition piece is then affixed to the proximal portion.

The transition piece may be adhesively bonded to the middle portion of the tip housing, in one example, or to the proximal portion of the tip housing, in another example. The transition piece may also be ultrasonically bonded, e.g fused.

As mentioned above, the tip housing 500b comprises at least three alignment surfaces 512. In a variation of the present embodiment, the at least three alignment surfaces 512 consist of two alignment surfaces 512rb and one anterior alignment surface 512ab. Alternatively, the at least three alignment surfaces 512 consist of two alignment surfaces 512rb and one posterior alignment surface 512pb.

In another variation of the present embodiment, the at least three alignment surfaces 512 consist of two alignment surfaces 512rb and two anterior alignment surfaces 512ab, one positioned distally of the other.

In a further variation of the present embodiment, the at least three alignment surfaces 12 consist of two alignment surfaces 512rb, two anterior alignment surfaces 512ab, and at least one posterior alignment surface 512pb.

In a yet further variation of the present embodiment, the at least three alignment surfaces 512 consist of two alignment surfaces 512rb, one anterior alignment surface 512ab, and one posterior alignment surface 512pb.

The working channel 600 comprises a distal working channel portion, or middle portion working channel, or working channel of the middle portion, 620 and a transition piece working channel 622. A longitudinal axis LA and the inlet hole 618 are shown in FIG. 16. The inlet hole 618 is shown at the juncture of the middle portion 512b and the transition piece 514b. FIG. 17 also shows a working channel axis WCA that is not concentric with the longitudinal axis LA, and a distal transition piece axis TPA(d) that is not parallel with the working channel axis WCA. An oval CS representing the cross-section of the working channel 600 is provided in FIG. 17 to illustrate the position of the longitudinal axis LA relative to the working channel axis WCA.

The transition piece working channel 622 comprises a distal working channel portion 624, a proximal working channel portion 626, and a wiring channel 632 formed between a circumferential wall 630 and a working channel wall 634. The proximal working channel portion 626 is offset from the middle portion working channel 620, their axes being parallel or not parallel. The proximal working channel portion 626 comprises a working channel wall 634 sized to receive a working channel tube 640. The distal transition piece axis TPA(d) is the longitudinal axis of the distal working channel portion 624. A proximal end of the transition piece axis TPA(p) is closer to the anterior channel 628 than a distal end thereof. In other words, the proximal working channel portion 626 of the transition piece is offset from the middle portion working channel 620. The transition piece working channel 622 may comprise a distal transition piece axis angle sufficiently large that a longitudinal projection of the camera module cavity 522b encompasses a working channel overlap portion 628 of the proximal working channel portion 626. A dashed line extending proximally from the working channel wall inner surface 608 is shown to better illustrate the working channel overlap portion 628.

By angling the transition piece working channel 622 it is possible to reduce the circumferential length, and the cross-section, of the tip housing 500b at its proximal end relative to its distal end. Thus, the tip housing 500b may comprise a distal cross section smaller than a proximal cross-section. Additionally, the transition piece with an angled working channel portion allows the working channel tube to be mounted in an unstressed state, which eases assembly. Angling the transition piece working channel 622 eases assembly as the working channel tube can be mounted straight onto the transition piece in its unbent natural state. The alternative would be to have the angling in the working channel tube itself, but bending the working channel tube, which is elastic/resilient, has the issue that the tube wants to bend back to its original unbent state. Trying to mount an angled tube that is trying to force itself back to its unbent state can be difficult, especially when trying to achieve a flush mounting to the tip housing middle working channel portion 620 to provide a transition that is as smooth as possible for passing tools. Furthermore, after mounting the working channel tube, the forces trying to get the tube back to its unbent state may unseat the working channel tube from the tip housing, causing misalignment or even a leak between the working channel tube and the tip housing.

The working channel tube 640 may be bent proximally of the tip housing 500b so that its longitudinal axis is closer to, or is parallel with, the longitudinal axis LA. The proximal working channel portion 626 may be angled at a proximal transition piece axis (TPA(p)) angle different than the distal transition piece axis angle. For example, the proximal transition piece axis angle may be smaller than the distal transition piece axis angle. This would permit a smaller amount of bending of the working channel tube 640. The angles of the proximal transition piece axis TPA(p) and the distal transition piece axis TPA(d) are measured relative to the working channel axis WCA.

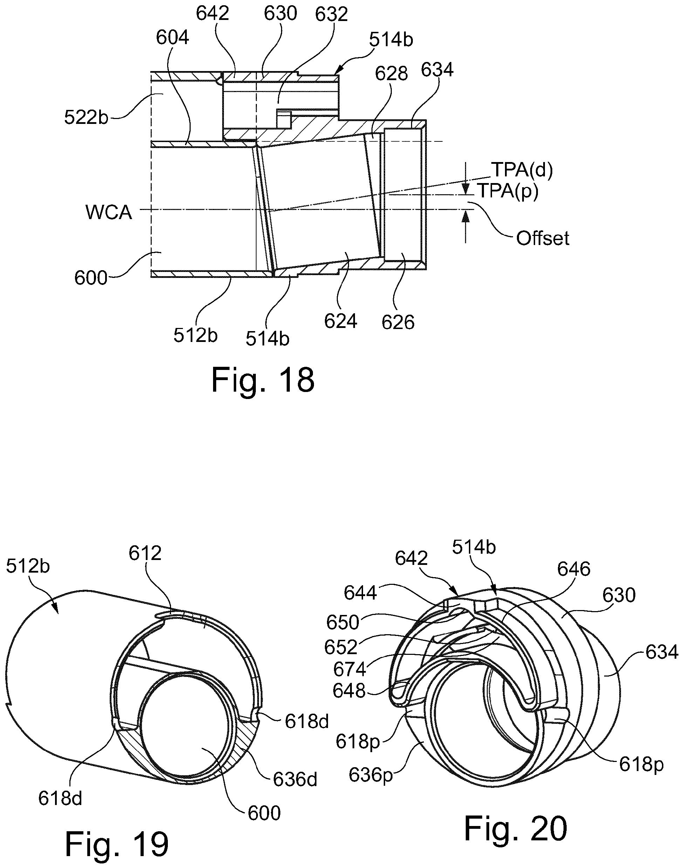

In another example, illustrated in FIGS. 18 to 20, the curvature that the working channel tube would have is incorporated in the tip housing 500b. An offset is present between the working channel axis WCA and the proximal transition piece axis TPA(p) which is parallel to the working channel axis WCA. The proximal transition piece axis TPA(p) is positioned between the anterior channel 632 and the working channel axis WCA. It should be noted that at least FIGS. 17 to 20 are drawn to scale, although not always to the same scale, for example to emphasize structural details. The transition piece 514b comprises a distal protrusion 642 that fits into the proximal end of the middle portion 512b and which is described in more detail below.

The working channel tube 640 may be inserted into the proximal working channel portion 626, which has a larger diameter than the distal working channel portion 624 for that purpose, and then adhesively bonded to the transition piece 514b. The diameters are sized to provide a seamless inner surface between the working channel tube 640 and the distal working channel portion 624. Thus, the diameter of the proximal working channel portion 626 might be larger than the diameter of the distal working channel portion 624 by twice the wall thickness of the working channel tube 640.

FIG. 19 shows the inlet notches 618d and a distal joint surface 636d that will be adhesively bonded to a proximal joint surface 636p of the transition piece 514b by injection of adhesive into the inlet holes 618. FIG. 20 shows the inlet notches 618p and the proximal joint surface 636p. The transition piece 514b comprises the distal protrusion 642 which is formed by a portion of the circumferential wall 630, denoted by numeral 646, and an anterior wall portion 648, at a distal end of the transition piece 514b. The anterior wall portion 648 is contoured to closely fit over the working channel wall 604. The thickness of the circumferential wall portion 646 is reduced relative to the thickness at the proximal end of the transition piece, except for an anterior peg 644 that has the same thickness as the proximal end. The peg 644 has a shape matching the notch 612 and fits in the notch 612. A longitudinal slot 650 extends along a posterior side of the anterior peg 644. A longitudinal slot 652 extends along an anterior side of the working channel wall 634. The slots 650 and 652 are aligned to provide additional space for wires along an anterior/posterior plane. Thus formed, the distal protrusion 642. The transition piece 514b in the present example may comprise a pair of steering wire holes positioned adjacent where the circumferential wall portion 646 and the anterior wall portion 648 adjoin. The transition piece 514b in the present example may also comprise a steering wire slot 674 extending between the pair of steering wire holes between the anterior wall portion 648 and the longitudinal slot 652.

In a variation of the present example, the notch 612 and the anterior peg 644 are omitted. The longitudinal slot 650 may also be omitted, depending on the size of the tip housing 500b. If the size is sufficiently large to allow the wires to pass without the longitudinal slot 650, the slot may be omitted and the circumferential wall portion 646 may thus be an arcuate portion that extends from side to side. Alternatively, an anterior cut-out may divide the circumferential wall portion 646 into two portions with a wire passage therebetween.

The following example of the tip housing 500b, illustrated in FIGS. 21 and 22, will further illustrate the steering wire slot 674 and the steering wire holes. In the present example, the middle section 512b comprises a cut-out 660 in the circumferential wall 518b that results in an arcuate portion 662 of the circumferential wall 518b extending proximally and over the distal end of the transition piece 514b to secure the transition piece 514b to the middle section 512b. The curvature that the working channel tube would have is also incorporated in the tip housing 500b. The offset is present between the working channel axis WCA and the proximal transition piece axis TPA(p). The inlet notches 618d are located at the juncture of the arcuate portion 662 and the uncut portion of the circumferential wall 518b. The circumferential wall 518b is devoid of the notch 612. The inlet notches 618d lie on a plane on which the proximal joint surface 636p also lies. The portion 646 of the circumferential wall 630 is also present although devoid of the anterior peg 644. The longitudinal slot 650 has been omitted in the present example, and the circumferential wall portion 646 is shown as an arcuate portion that extends from side to side. Alternatively, an anterior cut-out may divide the circumferential wall portion 646 into two portions with a wire passage therebetween.

A wall surface 672 of a transverse wall 670 extends between the portion 646 of the circumferential wall 630 and an arcuate steering wire slot 674 adjacent the working channel wall 634. At the ends of the arcuate steering wire slot 674, in the transverse wall 670, are a pair of steering wire holes 676. The circumferential wall 630 comprises a cut-out 678.

The bending section 32 comprises segments including a distal segment 700 and an intermediate segment 710. The segments are interconnected by living hinges 712 formed with the segments in a one-piece part. The living hinges 712 are bendable polymer strips that give the bending section at least some of its bending properties. The range of motion of the bending section may be defined by the shapes of the segments. The distal segment 700 comprises a wall 702 with a cut-out 704. A distal arcuate portion 706 of the wall 702 fits in the cut-out 678. An inlet hole 708 is provided to permit insertion of a liquid adhesive to bond the distal segment 700 to the insertion piece 514b. Generally, the cut-outs and remaining portions mate to interlock the parts in a more secure manner than if the parts were only adhesively bonded. Interlocking may be beneficial to add strength, particularly when the wall thicknesses are very small. Alternatively, the interlocking cut-outs may be omitted.

The intermediate segment 710 comprises steering wire holes 714 which are concentric with the steering wire holes 676 in the assembled state of the transition piece and the bending section. As shown in FIG. 22, the steering wire 42 comprises an intermediate portion 42a, which lies in the arcuate steering wire slot 674, and longitudinal portions 42b that extend from the intermediate portion 42a to the handle. To assemble the tip part 33b, the steering wire 42 is threaded through the steering wire holes 676 and then the steering wire holes 714 before the longitudinal portions 42b are passed through the insertion tube 31 and connected, directly or indirectly, to the steering actuator 25. In this manner the bending section 32 attaches to the tip assembly 33b and enables steering thereof via the steering actuator 25.

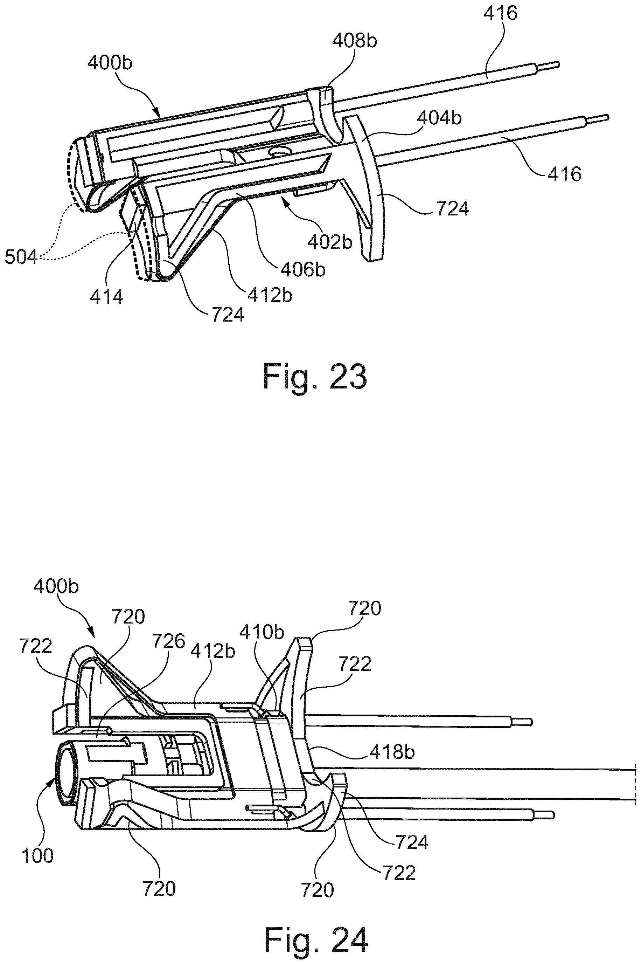

FIGS. 23 and 24 are two views of the second lighting module 400b. The second lighting module 400b comprises a body 402b including a proximal transverse wall 404b and a longitudinal bed 406b extending distally from the proximal transverse wall 404b. The proximal transverse wall 404b comprises a cable recess 408b configured to receive the cable assembly 110 or the wires thereof and/or the cable holder 203. At a distal end, the longitudinal bed 406b supports the LEDs 414 electrically connected to a circuit board 412b. Electrical lighting wires 416 electrically connect the LEDs 414, via the circuit board 412b, to a power source at the handle or at the video processor to power the LEDs. The proximal transverse wall 404b also comprises cable recesses 410b (FIG. 24) configured to receive the lighting wires 416 therethrough. As shown, the cable recess 408b is a cut-out and the cable recesses 410b are through-holes separated from the cable recess 408b by an intermediate portion 418b of the transverse wall 404b, and the longitudinal bed 406b extends longitudinally along a plane traversing the intermediate portion of the transverse wall 404b. The cable holder 203 may be translated longitudinally into the cable recess 408b or may be slid through the open end of the cut-out. Alternatively, the cable recess 408b may be a through-hole and, thus, the cable holder 203 may be translated longitudinally into the cable recess 408b. Dashed lines represent the lighting windows 504.

The second lighting module 400b may be electrically isolated, at the tip assembly, from the universal camera module 100.

Referring to FIG. 24, the second lighting module 400b further comprises four legs 720 extending posteriorly from the longitudinal bed 406b. Each of the legs 720 has an arcuate surface 722 facing toward the longitudinal axis LA and shaped with a contour matching the shape of the working channel wall 604. The opposite surface of each leg 720 has an arcuate surface 724 facing toward the circumferential wall 518b and shaped with a contour matching the shape of the wall inner surface 520b of the circumferential wall 518b. The longitudinal bed 406b comprises a cut-out 726 sized to receive a portion of the universal camera module 100 and thereby form a compact subassembly, comprising the universal camera module 100 and the second lighting module 400b, with reduced cross-section than if the cut-out 726 were not present. Alternatively, the thickness of the section corresponding to the cut-out 726 can be reduced instead of being entirely eliminated via the cut-out. For clarity, a cut-out refers a shape with a portion removed. Thus, the longitudinal bed 406b would comprise a generally rectangular shape without the cut-out.

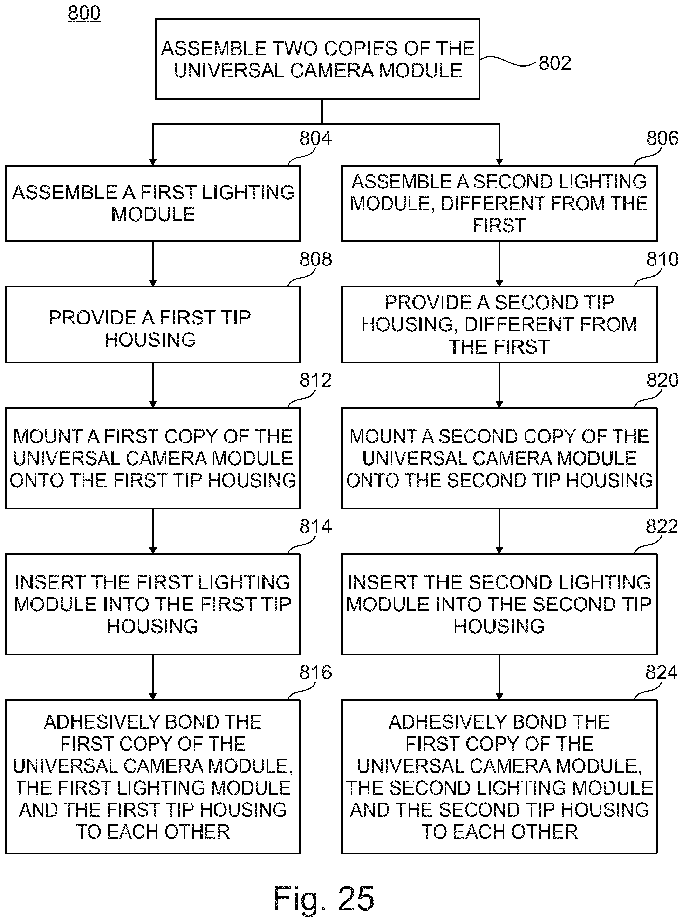

A method of making endoscopes for the system depicted with reference to FIGS. 2 to 6 will now be described with reference to a flowchart 800 in FIG. 25. In an embodiment of the method of making endoscopes, the method comprises:
at 802, assembling a first copy of a universal camera module 100 and a second copy of the universal camera module 100, the first copy being identical to the second copy;
at 804, assembling a first lighting module 400a;
at 806, assembling a second lighting module 400b different than the first lighting module 400a;
at 808, providing a first tip housing 500a;
at 810, providing a second tip housing 500b different than the first tip housing 500a;
at 812, inserting the first copy of the universal camera module 100 into the first tip housing 500a;
at 814, inserting the first lighting module 400a into the first tip housing 500a;
at 816, adhesively bonding the first copy of the universal camera module 100, the first lighting module 400a and the first tip housing 500a to each other;
at 820, inserting the second copy of the universal camera module 100 into the second tip housing 500b;
at 822, inserting the second lighting module 400b into the second tip housing 500b; and
at 824, adhesively bonding the second copy of the universal camera module 100, the second lighting module 400b and the second tip housing 500b to each other.

The universal camera modules 100 and the lighting modules 400a, 400b can be assembled at any time prior to mounting the universal camera modules on the tip housings, in any order. The tip housings may be provided at any time prior to insertion of the subassemblies in them. The universal camera module 100 and the lighting module 400a, 400b may be inserted into the tip housing 500a, 500b, respectively, at the same time and then the universal camera module 100 may be pressed until distal translation ceases due to contact with the housing. The lighting module 400a, 400b can be pressed until the LED emitting surface abuts the lighting window or the light guide. Movement of the lighting module 400a, 400b into position is independent of movement of the universal camera module 100 into its inserted position. After insertion the universal camera module 100 may be adhesively fixated to the distal end of the tip housing 500a, 500b before the lighting module 400a, 400b is inserted into the tip housing 500a, 500b. Then, the universal camera module 100, the lighting module 400a, 400b and the tip housing 500a, 500b may be adhesively bonded to each other by inserting glue through the proximal end of the tip housing 500a, 500b.

In a variation of the present embodiment, the second tip housing 500b comprises a working channel outlet and the first tip housing 500a is devoid of a working channel outlet.

In another variation of the aforementioned embodiment of a method of making endoscopes, the universal camera module comprises first housing alignment features and second housing alignment features different from the first housing alignment features, the first housing alignment features abut alignment surfaces of the first tip housing to align the universal camera module in the first tip housing, and the second housing alignment features abut alignment surfaces of the second tip housing align the universal camera module in the second tip housing.

The first lighting module 400a may be inserted into the first tip housing 500a concurrently with the universal camera module 100. Distal movement of the lighting module 400a, 400b stops when the LED 414 abuts the lighting window 504. Alternatively, a light guide may be provided and in that case distal movement of the lighting module 400a, 400b stops when the LED 414 abuts the light guide. Distal movement of the universal camera module 100 may cease when the front end of the universal camera module 100 abuts the viewing window 502.

Referring now to FIG. 26, a light guide 840 is interposed between the LED 414 and the lighting window 504. The light guide 840 and the lighting window 504 may be molded as one-piece part and may molded together with the tip housing 500. The LED 414, as is known in the art, comprises a substrate 414a with a light emission surface 414b and a pair of electrical contacts 414c. The light emission surface 414b faces a light reception surface of the light guide 840. The light guide 840 is shaped and, potentially, cladded to provide total internal reflection and shape the light emitted by the LED 414 into a desired light pattern based on the position of the light guide 840 and the universal camera module 100 to illuminate a desired field-of-view of the universal camera module 100. The light travels through the light guide 840 and into the lighting window 504 without a transition therebetween, therefore it can be considered that the light guide extends through the lighting window 504 and the light emission surface of the light guide 840 is, therefore, the outer surface of the lighting window 504. As can be seen, the light guide in the present example is a truncated pyramid with a generally rectangular cross-section, which has been found useful for a rectangular image sensor 102. Alternatively, the light guide can be a separate part that is adhesively bonded to the lighting window 504.

The aforementioned embodiment of the method of making endoscopes may further comprise threading the camera module wires 111, and/or the cable assembly 110, and the lighting wires 416 through the insertion tube 31 of the endoscope 20.

An endoscope according to the second aspect, made in accordance with the method described above with reference to FIG. 25, will now be described. In one embodiment, the endoscope comprises:
- a positioning interface or handle 21;
- an insertion tube 31 extending distally from the positioning interface or handle 21;
- a lighting module 400a, 400b comprising a body 402 including a longitudinal bed 406, 406b and a transverse wall 404, 404b, a light emitting diode 414 supported at a distal end of the longitudinal bed 406, 406b, and lighting wires 416 electrically connected to the light emitting diode 414 and extending through the insertion tube 31 to the positioning interface or handle 21;
- a universal camera module 100 comprising an image sensor 102, a circuit board 103 electrically connected to the image sensor 102, a sensor holder 200, and a sensor housing 300 affixed to the sensor holder 200, the image sensor 102 housed in the sensor housing 300, and the sensor holder 200 extending proximally from the image sensor 102;
- a tip housing 500a, 500b comprising a sensor module cavity 524, 524b configured to receive a distal end of the sensor housing 300 and an LED cavity 526, 526b configured to receive the LEDs of the lighting module 400a, 400b, wherein the universal camera module 100, the lighting module 400a, 400b and the tip housing 500a, 500b are adhesively bonded; and
- camera module wires 111 electrically connected to the circuit board 103 and extending through the insertion tube 31 to the positioning interface or handle 21.

The universal camera module 100 and the lighting module 400a, 400b can be adhesively bonded, separately, to the tip housing 500a, 500b. Additionally, they can also be adhesively bonded to each other, after distal movement ceased and they are in their final position, and to the tip housing 500a, 500b.

FIGS. 27 and 28 illustrate two embodiments of the video processor 11, denoted by numerals 11a and 11b. The video processor 11 comprises a cable socket 850 to which the video conector 24 of the endoscope 20 (shown in FIG. 1) can be connected to establish signal communication between the image sensor 102 and the video processor 11 via the cable 23 and wires 111. The video processor 11 is configured to cause a display to present images and/or video captured by the image sensor 102 thus allowing an operator to "see" a body cavity into which the endoscope 20 has be inserted.

The video processor 11 comprises a housing 852, one or more medical device interfaces connected to the cable socket(s) 850, and a video processing circuit (not shown). Variations of the video processor 11 can be provided. For example, it might not be desirable to provide a display screen with a touch screen (e.g. video processor 11a comprising display screen 854), or it might be desirable to omit a display screen altogether (e.g. video processor 11b). Omission of the display screen might be beneficial to take advantage of evolving video display technologies which improve resolution and reduce cost. Provision of exchangeable medical device interfaces allows for adoption of evolving image sensor and endoscope technologies, thus use of existing or future-developed external video displays could allow presentation of higher resolution or otherwise improved video. Use of external video displays could also leverage existing capital investments.

The video processor 11a comprises a housing 852, a display screen 854, and one or more medical device interfaces connected to the cable socket(s) 850.

In all embodiments and variations thereof, the endoscope may be disposable and may not be intended to be cleaned and reused. Alternatively, the endoscope may, in all embodiments, be re-usable. In some variations of the present embodiment, the endoscope and the video processor comprise wireless transceivers to exchange image data and configuration data. The endoscope may comprise a battery to power the image sensor and light sources, such as light emitting diodes (LEDs).

The video processing circuit of the video processor is operable to receive image data, present a graphical user interface to allow a user to manipulate image data with the touch screen, and, optionally, output a video signal to allow remote viewing of the images presented with the display screen. A separate, potentially remote, display screen may also be connected to the endoscope via the video processor, which may include or omit the display screen. The medical device interfaces include circuits to compatibilize the signals from the image sensors, for example. Thus, a particular type of endoscope is matched with a corresponding medical device interface and the video processorcan thus enable use of different endoscope or other medical device technologies. In other words, the video processoror monitor is customized to work with the particular endoscope's technology. The medical device interfaces may also include isolation amplifiers to electrically isolate the video signal, and a power ouput connector to provide power to the endoscope for the image sensor and the LEDs. The medical device interfaces may also include a serial to parallel converter circuit to deserialize the video signals of endoscopes that generate serial signals, for example serial analog video signals. The medical device interfaces may also include a configuration connector to output image sensor configuration parameters such as image inversion, clock, shutter speed etc.

A positioning interface, or interface, functions to control the position of the insertion cord 30. The handle 21 is an example of a positioning interface and, unless stated otherwise, the terms are used interchangeably. The positioning interface also functions to provide the steering controls, e.g. knobs, levers, buttons, and the like, to steer the field of view of the camera. Alternatively, a different positioning interface can be provided that is connected to the insertion cord and is detachably connected to a robotic arm. The insertion cord thus extends from the robotic arm, and the intrusive medical device is detachable from the robotic arm. The robotic arm responds to signals, including voice commands from an operator, to rotate, translate, and otherwise position the proximal end of the insertion cord, as an operator would do manually. The positioning interface can include control actuators, including manual control actuators. Alternatively or additionally, control actuators can be provided in or on the robotic arm or by the robotic system including the robotic arm, thereby potentially reducing the cost of the intrusive medical device. Example control actuators include single axis actuators, including linear motion actuators. A linear motion actuator may comprise a threaded rod coupled to a threaded nut portion, in which a motor rotates the rod to translate the nut portion.

The following items are further variations and examples of the embodiments described with reference to the figures.
1. A method of making endoscopes, the method comprising: assembling a first copy of a universal camera module (100) and a second copy of the universal camera module (100), the first copy being identical to the second copy; assembling a first lighting module (400a); assembling a second lighting module (400b) different than the first lighting module (400a); providing a first tip housing (500a); providing a second tip housing (500b) different than the first tip housing (500a); inserting the first copy of the universal camera module (100) and the first lighting module (400a) into the first tip housing (500a) to form a first tip assembly (33a); and inserting the second copy of the universal camera module (100) and the second lighting module (400b) into the second tip housing (500b) to form a second tip assembly (33b).
2. The method of item 1, wherein the universal camera module (100) comprises first anti-rotation surfaces (312r) and second anti-rotation surfaces (312la) different from the first anti-rotation surfaces (312r), wherein the first tip housing (500a) comprises first tip housing anti-rotation surfaces (512r) and the second tip housing (500b) comprises second tip housing anti-rotation surfaces (512la), and wherein: inserting the first copy of the universal camera module (100) comprises translating the first copy of the universal camera module (100) to abut the first anti-rotation surfaces (312r) and the first tip housing anti-rotation surfaces (512r), and inserting the second copy of the universal camera module (100) comprises translating the second copy of the universal camera module (100) to abut the second anti-rotation surfaces (312la) and the second tip housing anti-rotation surfaces (512la).
3. The method of item 2, wherein the universal camera module (100) comprises wings (320) extending longitudinally, and wherein the wings (320) comprise the first anti-rotation surfaces (312r) and second anti-rotation surfaces (312la).
4. The method of item 3, wherein the first anti-rotation surfaces (312r) are posterior surfaces of the wings (320) and the second anti-rotation surfaces (312la) are lateral surfaces of the wings (320).
5. The method of item 4, wherein the wings (320) extend laterally from a circumferential wall (302) of the sensor housing (300).
6. The method of any one of items 1-5, further comprising inserting an image sensor (102) into a proximal end of a sensor housing (300) and adhesively bonding a sensor holder (200) and the sensor housing (300) with the image sensor (102) housed in the sensor housing (300).
7. The method of item 6, wherein the camera housing (300) and/or the sensor holder (200) comprises arms (340) extending between the camera housing (300) and the sensor holder (200) around a portion of a circuit board (103) electrically connected to an image sensor (102), the method further comprising adhesively bonding the arms (340) to the camera housing (300) and/or the sensor holder (200) to affix the sensor holder (200) and the camera housing (300) to each other.
8. The method of item 6, wherein the circuit board (103) is electrically connected to camera module wires (111), wherein the first lighting module (400a) comprises a light emitting diode (414) and lighting wires (416) electrically connected to the light emitting diode (414), the method further comprising threading the camera module wires (111) and the lighting wires (416) through an insertion tube of the endoscope (20).
9. The method of item 8, wherein the first lighting module (400a) is electrically isolated, at the first tip assembly (33a), from the universal camera module (100).
10. The method of any one of items 1-9, wherein the first tip housing (500a) comprises a lighting window (504) and a light receiving surface (504p), and wherein inserting the first copy of the universal camera module (100) and the first lighting module (400a) into the first tip housing (500a) to form the first tip assembly (33a) comprises ceasing to distally translate the first lighting module (400a) when a light emitting diode (414) of the first lighting module (400a) abuts the light receiving surface (504p) and ceasing to distally translate the universal camera module (100) when the universal camera module (100) abuts a proximally-facing surface of the first tip housing (500a), said ceasing to distally translate the first lighting module (400a) and said ceasing to distally translate the universal camera module (100) occurring sequentially.
11. The method of any one of items 1-10, wherein the second tip housing (500b) comprises a working channel (600) and the first tip housing (500a) is devoid of a working channel.
12. The method of item 11, wherein the second tip housing (500b) comprises a middle portion (512b), and a transition portion (514b), the transition portion (514b) comprising a distal portion (624) with a longitudinal axis and a proximal portion (626) with a longitudinal axis offset from the longitudinal axis of the distal portion (624), the method further comprising inserting a distal end of a working channel tube (640) into the proximal portion (626).
13. The method of item 12, the method further comprising adhesively bonding the transition portion (514b) to the middle portion (512b).
14. The method of any of the preceding items, wherein the first lighting module (400a) comprises a body (402) including a longitudinal bed (406) and a transverse wall (404), the longitudinal bed (406) comprises a distal bed portion (406d), a proximal bed portion (406p), and a step (420) that offsets, longitudinally, the distal bed portion (406d) and the proximal bed portion (406p), the transverse wall (404) comprises a posterior portion (404p) extending posteriorly from the proximal bed portion (406p) and comprising a cable recess (410, 410b), and the transverse wall (404) further comprises a cable recess (408) positioned anteriorly from the proximal bed portion (406p), wherein the sensor holder (200) comprises a cable holder (202), and wherein the method further comprises positioning the cable holder (202), at least in part, in the cable recess (408).
15. The method of any of the preceding items, the method further comprising: attaching the first tip assembly (33a) to a first bending section (32), attaching the first bending section (32) to a first insertion tube (31), and attaching the first insertion tube (31) to a first handle or positioning interface (21) to assemble a first endoscope (20), and attaching the second tip assembly (33b) to a second bending section (32), attaching the second bending section (32) to a second insertion tube (31), and attaching the second insertion tube (31) to a second handle or positioning interface (21) to assemble a second endoscope (20).
16. An endoscope (20) as in any of the following items, manufacturing according to the method recited in any one of items 1-15.
21. An endoscope (20) comprising: a positioning interface or handle (21); an insertion tube extending distally from the positioning interface or handle (21); a lighting module (400, 400a, 400b) comprising a body (402) including a longitudinal bed (406) and a transverse wall (404), a light emitting diode (414) supported at a distal end of the longitudinal bed (406), and lighting wires (416) electrically connected to the light emitting diode (414); a universal camera module (100) comprising an image sensor (102), circuit board electrically connected to the image sensor (102), a sensor holder, and a sensor housing affixed to the sensor holder, the image sensor (102) housed in the sensor housing, and the sensor holder extending proximally from the image sensor (102); camera module wires (111) electrically connected to the circuit board (103) electrically connected to the image sensor (102); and a tip housing (500, 500a, 500b) enclosing the lighting module (400, 400a, 400b) and the universal camera module (100).
22. The endoscope (20) of item 21, wherein the lighting wires extend through the insertion tube to the positioning interface or handle (21), and wherein the camera module wires extend through the insertion tube to the positioning interface or handle (21).
23. The endoscope (20) of item 21, wherein the longitudinal bed (406) comprises a distal bed portion (206d), a proximal bed portion (206p), and a step (420) that offsets, longitudinally, the distal bed portion (206d) and the proximal bed portion (206p).
24. The endoscope (20) of item 21, wherein the longitudinal bed (406) comprises a distal bed portion (406d), a proximal bed portion (406p), and a step (420) that offsets, longitudinally, the distal bed portion (406d) and the proximal bed portion (406p), the transverse wall (404) comprising a posterior portion (404p) extending posteriorly from the proximal bed portion (406p) and comprising a cable recess (410, 410b), with the lighting wires (416) traversing the cable recess (410, 410b).
25. The endoscope (20) of item 34, wherein the transverse wall (404) further comprises a cable recess (408) positioned anteriorly of the proximal bed portion (406p), wherein the sensor holder (200) comprises a cable holder (202), wherein the cable holder (202) is positioned, at least in part, in the cable recess (408), and wherein the camera module wires extend through the cable recess (408).
26. The endoscope (20) of any one of items 21-25, wherein the universal camera module (100) is electrically isolated from the lighting module 400a, 400b at the tip housing (33a, 33b).
27. The endoscope (20) of any one of items 21-26, wherein the sensor housing (300) comprises a circumferential wall (302) and wings (320) that extend laterally from the circumferential wall (302), the wings (320) comprising first anti-rotation surfaces (312r) and second anti-rotation surfaces (312la) different from the first anti-rotation surfaces (312r), the tip housing (500a, 500b) comprising first tip housing anti-rotation surfaces (512r) or second tip housing anti-rotation surfaces (512rb), the first anti-rotation surfaces (312r) abutting the first tip housing anti-rotation surfaces (512r) or the second anti-rotation surfaces (312r) abutting the second tip housing anti-rotation surfaces (512la).
28. The endoscope (20) of item 27, wherein the image sensor (102) is positioned in a proximal end of the sensor housing (300) and the sensor holder (200) is adhesively bonded to the sensor housing (300).
29. The endoscope (20) of item 27, wherein the image sensor (102) is positioned in a proximal end of the sensor housing (300), wherein a distal end of the circuit board (103) is attached to the image sensor (102) and comprises recesses (103r), the sensor housing (300) and/or the sensor holder (200) comprises arms (340) extending between the sensor housing (300) and/or the sensor holder (200), and wherein the arms (340) traverse the recesses (103r) and affix the sensor holder (200) and the camera housing (300) to each other.
30. The endoscope (20) of item 29, wherein the arms (340) extend proximally from the wings (320).
31. The endoscope (20) of any of the preceding items, wherein the tip housing (500b) comprises a working channel (600), a middle portion (512b), and a transition portion (514b), the working channel (600) extending through the middle portion (512b) and the transition portion (514b), the transition portion (514b) comprising a distal portion with a longitudinal axis and a proximal portion (626) with a longitudinal axis offset from the longitudinal axis of the distal portion (624), the endoscope (20) further comprising a working channel tube (640) affixed to the proximal portion.
32. The endoscope (20) of item 31, wherein the tip housing (500, 500a, 500b) comprises a camera module cavity (522, 522b), wherein the transition piece comprises a distal protrusion (642) that fits in the camera module cavity (522, 522b) and a proximal joint surface (636p), and wherein the middle portion (512b) comprises a distal joint surface (636d) adhesively bonded to the proximal joint surface (636p) forming a joint (118), wherein the distal protrusion (642) extends distally of the joint (118).
33. The endoscope (20) of item 31, wherein the middle portion (512b) comprises a proximal joint surface (636p), wherein the middle portion (512b) comprises a circumferential wall (518b) comprising an arcuate portion (662), wherein the transition piece comprises a distal joint surface (636p) and a circumferential wall portion (646) positioned anteriorly of the transition piece working channel (622), wherein the distal joint surface (636d) is adhesively bonded to the proximal joint surface (636p) forming a joint (118), and wherein the arcuate portion (662) extends over the joint (118).
34. The endoscope (20) of item 31, wherein the middle portion (512b) comprises a proximal joint surface (636p), wherein the transition piece (514b) comprises a distal joint surface (636p), wherein the distal joint surface (636d) is adhesively bonded to the proximal joint surface (636p) forming a joint (118), and wherein the transition piece (514b) comprises an arcuate steering wire slot (674) positioned distally of the proximal working channel portion (626).
41. An endoscope (20) comprising: a positioning interface or handle (21); an insertion tube extending distally from the positioning interface or handle (21); a lighting module (400b) comprising a light emitting diode (414); a universal camera module (100) comprising an image sensor (102), circuit board electrically connected to the image sensor (102), and a sensor housing, the image sensor (102) housed in the sensor housing; and a tip housing (500b) enclosing the lighting module (400b) and the universal camera module (100), the tip housing (500b) comprising a working channel (600), a distal portion (51 0b), a transition piece (514b) and a middle portion (512b) positioned between the distal portion (510b) and the transition piece (514b), the middle portion (512b) being opaque and the distal portion (510b) being transparent, the working channel (600) comprising a middle portion working channel (620) and a transition piece working channel (622), wherein the middle portion (512b) comprises the middle portion working channel (620), and wherein the transition piece (514b) comprises the transition piece working channel (622), the transition piece working channel (622) including a distal working channel portion (624) distal of a proximal working channel portion (626), the proximal working channel portion (626) being offset from the middle portion working channel (620).
42. The endoscope (20) of item 41, wherein the middle portion working channel (620) comprises a longitudinal axis (WCA), and wherein the proximal working channel portion (626) comprises a longitudinal axis (TPA(p)) that is parallel to the longitudinal axis of the middle portion working channel (620).
43. The endoscope (20) of item 42, wherein the transition piece (514b) is transparent and is adhesively bonded to the middle portion.
44. The endoscope (20) of item 41, wherein the tip housing comprises a camera module cavity (522b), wherein the transition piece comprises a distal protrusion (642) that fits in the camera module cavity (522b) and a proximal joint surface (636p), and wherein the middle portion (512b) comprises a distal joint surface (636d) adhesively bonded to the proximal joint surface (636p) forming a joint (118), wherein the distal protrusion (642) extends distally of the joint (118).
45. The endoscope (20) of item 41, wherein the middle portion (512b) comprises a proximal joint surface (636p), wherein the middle portion (512b) comprises a circumferential wall 518b comprising an arcuate portion (662), wherein the transition piece comprises a distal joint surface (636p) and a circumferential wall portion (646) positioned anteriorly of the transition piece working channel (622), wherein the distal joint surface (636d) is adhesively bonded to the proximal joint surface (636p) forming a joint (118), and wherein the arcuate portion (662) extends over the joint (118).
46. The endoscope (20) of item 41, wherein the middle portion (512b) comprises a proximal joint surface (636p), wherein the transition piece (514b) comprises a distal joint surface (636p), wherein the distal joint surface (636d) is adhesively bonded to the proximal joint surface (636p) forming a joint (118), and wherein the transition piece (514b) comprises an arcuate steering wire slot (674) positioned distally of the proximal working channel portion (626).
47. The endoscope (20) of item 41, wherein the lighting wires (616) extend through the insertion tube (31) to the positioning interface or handle (21), and wherein the camera module wires (111) extend through the insertion tube (31) to the positioning interface or handle (21).
48. The endoscope (20) of item 41, wherein the longitudinal bed (406) comprises a distal bed portion (206d), a proximal bed portion (206p), and a step (420) that offsets, longitudinally, the distal bed portion (206d) and the proximal bed portion (206p).
49. The endoscope (20) of item 41, wherein the longitudinal bed (406) comprises a distal bed portion (406d), a proximal bed portion (406p), and a step (420) that offsets, longitudinally, the distal bed portion (406d) and the proximal bed portion (406p), the transverse wall (404) comprising a posterior portion (404p) extending posteriorly from the proximal bed portion (406p) and comprising a cable recess (410b), with the lighting wires (416) traversing the cable recess (410b).
50. The endoscope (20) of item 49, wherein the transverse wall (404) further comprises a cable recess (408) positioned anteriorly of the proximal bed portion (406p), wherein the sensor holder (200) comprises a cable holder (202), wherein the cable holder (202) is positioned, at least in part, in the cable recess (408), and wherein the camera module wires extend through the cable recess (408).
51. The endoscope (20) of any one of items 41-50, wherein the universal camera module (100) is electrically isolated from the lighting module (400b) at the first tip assembly (33b).
52. The endoscope (20) of any one of items 41-50, wherein the sensor housing comprises a circumferential wall (302) and wings (320) that extend laterally from the circumferential wall (302), the wings (320) comprising anti-rotation surfaces (312la), the tip housing (500b) comprising tip housing anti-rotation surfaces (512rb), the anti-rotation surfaces (312la) abutting the tip housing anti-rotation surfaces (512la).
53. The endoscope (20) of item 52, wherein the image sensor (102) is positioned in a proximal end of the sensor housing (300) and the sensor holder (200) is adhesively bonded to the sensor housing (300).
54. The endoscope (20) of item 52, wherein the image sensor (102) is positioned in a proximal end of the sensor housing (300), wherein a distal end of the circuit board (103) is attached to the image sensor (102) and comprises recesses (103r), the sensor housing (300) and/or the sensor holder (200) comprises arms (340) extending between the sensor housing (300) and/or the sensor holder (200), and wherein the arms (340) traverse the recesses (103r) and affix the sensor holder 200 and the camera housing (300) to each other.
55. The endoscope (20) of item 54, wherein the arms (340) extend proximally from the wings (320).

### Parts list:

- 10: visualization system
- 11, 11a, 11b: video processor
- 20: endoscope
- 21: handle or positioning interface
- 22: housing
- 23: wire cord
- 24: connector
- 25: steering actuator
- 30: insertion cord
- 31: insertion tube
- 32: bending section
- 33, 33a, 33b: tip assembly
- 40: working channel
- 41: tool
- 42: steering wire
- 42a: intermediate portion
- 42b: longitudinal portions

- 100: universal camera module
- 102: image sensor
- 103: circuit board
- 103r: recess
- 104: circuit board
- 110: cable assembly
- 111: camera module wires
- 112: electrical shield
- 113: cable cover
- 118: joint

- 200: sensor holder
- 202: cable holder
- 203: outer surface
- 204: recess
- 206: longitudinal bed
- 206d: distal portion of the longitudinal bed
- 206p: proximal portion of the longitudinal bed

- 300: sensor housing
- 302: circumferential wall
- 304: sensor cavity
- 306: lenses
- 312, 312a, 312ap: alignment surfaces
- 312la, 312r: alignment/anti-rotation surfaces
- 320: wings
- 340: arm
- 400, 400a, 400b: lighting module
- 402, 402b: body
- 404, 404b: transverse wall
- 404p: posterior side of the transverse wall
- 406, 406b: longitudinal bed
- 406d: distal portion of the longitudinal bed
- 406p: proximal portion of the longitudinal bed
- 408, 408b: cable recess
- 410, 410b: cable recess
- 412, 412b: circuit board
- 414: light emitting diodes (LEDs)
- 414a: substrate
- 414b: light emission surface
- 414c: electrical contacts
- 416: lighting wires
- 418: intermediate portion
- 420: step

- 500a, 500b: tip housing
- 502: viewing window
- 504: lighting window
- 504p: light receiving surface
- 510, 510b: distal portion
- 512, 512b: middle portion
- 512a, 512p: alignment surface
- 512ab, 512pb: alignment surface
- 512r, 512rb: anti-rotation surface
- 514, 514b: proximal portion /transition piece

- 518, 518b: circumferential wall
- 519, 519b: outer surface of the circumferential wall
- 520, 520b: inner surface of the circumferential wall
- 522, 522b: camera module cavity
- 524, 524b: sensor module cavity
- 526, 526b: LED cavity
- 528, 528b: intermediate wall

- 600: working channel
- 604: working channel wall
- 606: working channel wall outer surface
- 608: working channel wall inner surface
- 610: housing portion
- 612: anterior notch
- 618: holes 618
- 618d: inlet notches
- 618p: inlet notches
- 620: working channel of middle portion
- 622: working channel of transition piece
- 624: distal working channel portion of the transition piece
- 626: proximal working channel portion of the transition piece
- 628: anterior channel
- 630: circumferential wall
- 632: a wiring channel of the transition piece
- 634: working channel wall
- 636d: distal joint surface
- 636p: proximal joint surface
- 640: working channel tube
- 642: the distal protrusion
- 644: anterior peg
- 646: portion of the circumferential wall
- 648: anterior wall portion
- 650: longitudinal slot
- 652: longitudinal slot
- 662: arcuate portion
- 670: transverse wall
- 672: wall surface
- 674: steering wire slot
- 676: steering wire holes
- 678: cut-out

- 700: distal segment
- 702: wall
- 704: cut-out
- 706: distal arcuate portion of the wall
- 708: inlet hole
- 710: intermediate segment
- 712: living hinges
- 714: steering wire holes
- 720: leg
- 722: arcuate surface
- 724: arcuate surface
- 726: cut-out
- 800: flowchart
- 840: light guide
- 850: cable socket(s)
- 852: housing
- 854: display screen

- CS: oval, cross-section of the working channel
- LA: longitudinal axis
- TPA(d): distal transition piece axis
- WCA: working channel axis

## Claims

1. An endoscope (20) comprising:
a positioning interface or handle (21);
an insertion tube extending distally from the positioning interface or handle (21);
a lighting module (400, 400a, 400b) comprising a body (402) including a longitudinal bed (406) and a transverse wall (404), a light emitting diode (414) supported at a distal end of the longitudinal bed (406), and lighting wires (416) electrically connected to the light emitting diode (414);
a universal camera module (100) comprising an image sensor (102), circuit board electrically connected to the image sensor (102), a sensor holder, and a sensor housing affixed to the sensor holder, the image sensor (102) housed in the sensor housing, and the sensor holder extending proximally from the image sensor (102);
camera module wires (111) electrically connected to the circuit board (103) electrically connected to the image sensor (102); and
a tip housing (500, 500a, 500b) enclosing the lighting module (400, 400a, 400b) and the universal camera module (100).

2. The endoscope (20) of claim 1, wherein the lighting wires extend through the insertion tube to the positioning interface or handle (21), and wherein the camera module wires extend through the insertion tube to the positioning interface or handle (21).

3. The endoscope (20) of claim 1, wherein the longitudinal bed (406) comprises a distal bed portion (206d), a proximal bed portion (206p), and a step (420) that offsets, longitudinally, the distal bed portion (206d) and the proximal bed portion (206p).

4. The endoscope (20) of claim 1, wherein the longitudinal bed (406) comprises a distal bed portion (406d), a proximal bed portion (406p), and a step (420) that offsets, longitudinally, the distal bed portion (406d) and the proximal bed portion (406p), the transverse wall (404) comprising a posterior portion (404p) extending posteriorly from the proximal bed portion (406p) and comprising a cable recess (410, 410b), with the lighting wires (416) traversing the cable recess (410, 410b).

5. The endoscope (20) of claim 4, wherein the transverse wall (404) further comprises a cable recess (408) positioned anteriorly of the proximal bed portion (406p), wherein the sensor holder (200) comprises a cable holder (202), wherein the cable holder (202) is positioned, at least in part, in the cable recess (408), and wherein the camera module wires extend through the cable recess (408).

6. The endoscope (20) of any one of claims 1-5, wherein the universal camera module (100) is electrically isolated from the lighting module 400a, 400b at the tip housing (33a, 33b).

7. The endoscope (20) of any one of claims 1-6, wherein the sensor housing (300) comprises a circumferential wall (302) and wings (320) that extend laterally from the circumferential wall (302), the wings (320) comprising first anti-rotation surfaces (312r) and second anti-rotation surfaces (312la) different from the first anti-rotation surfaces (312r), the tip housing (500a, 500b) comprising first tip housing anti-rotation surfaces (512r) or second tip housing anti-rotation surfaces (512rb), the first anti-rotation surfaces (312r) abutting the first tip housing anti-rotation surfaces (512r) or the second anti-rotation surfaces (312r) abutting the second tip housing anti-rotation surfaces (512rb).

8. The endoscope (20) of claim 7, wherein the image sensor (102) is positioned in a proximal end of the sensor housing (300) and the sensor holder (200) is adhesively bonded to the sensor housing (300).

9. The endoscope (20) of claim 7, wherein the image sensor (102) is positioned in a proximal end of the sensor housing (300), wherein a distal end of the circuit board (103) is attached to the image sensor (102) and comprises recesses (103r), the sensor housing (300) and/or the sensor holder (200) comprises arms (340) extending between the sensor housing (300) and/or the sensor holder (200), and wherein the arms (340) traverse the recesses (103r) and affix the sensor holder (200) and the camera housing (300) to each other.

10. The endoscope (20) of claim 9, wherein the arms (340) extend proximally from the wings (320).

11. The endoscope (20) of any of the preceding claims, wherein the tip housing (500b) comprises a working channel (600), a middle portion (512b), and a transition portion (514b), the working channel (600) extending through the middle portion (512b) and the transition portion (514b), the transition portion (514b) comprising a distal portion with a longitudinal axis and a proximal portion (626) with a longitudinal axis offset from the longitudinal axis of the distal portion (624), the endoscope (20) further comprising a working channel tube (640) affixed to the proximal portion.

12. The endoscope (20) of claim 11, wherein the tip housing (500, 500a, 500b) comprises a camera module cavity (522, 522b), wherein the transition piece comprises a distal protrusion (642) that fits in the camera module cavity (522, 522b) and a proximal joint surface (636p), and wherein the middle portion (512b) comprises a distal joint surface (636d) adhesively bonded to the proximal joint surface (636p) forming a joint (118), wherein the distal protrusion (642) extends distally of the joint (118).

13. The endoscope (20) of claim 11, wherein the middle portion (512b) comprises a proximal joint surface (636p), wherein the middle portion (512b) comprises a circumferential wall (518b) comprising an arcuate portion (662), wherein the transition piece comprises a distal joint surface (636p) and a circumferential wall portion (646) positioned anteriorly of the transition piece working channel (622), wherein the distal joint surface (636d) is adhesively bonded to the proximal joint surface (636p) forming a joint (118), and wherein the arcuate portion (662) extends over the joint (118).

14. The endoscope (20) of claim 11, wherein the middle portion (512b) comprises a proximal joint surface (636p), wherein the transition piece (514b) comprises a distal joint surface (636p), wherein the distal joint surface (636d) is adhesively bonded to the proximal joint surface (636p) forming a joint (118), and wherein the transition piece (514b) comprises an arcuate steering wire slot (674) positioned distally of the proximal working channel portion (626).
